# EUROPEAN PATENT APPLICATION

(11) **EP 4 692 111 A1**
(43) Date of publication of application: **11.02.2026**
(21) Application number: 24788022.2
(22) Date of filing: 08.04.2024
(51) Int. Cl.: C07K 14/01, C07K 19/00, A61K 39/12, A61K 39/295, A61P 31/20

(54) **SEVEN-COMPONENT ANTIGEN AFRICAN SWINE FEVER SUBUNIT VACCINE**

(30) Priority: 10.04.2023 CN 202310373357
(71) Applicant: Lanzhou Veterinary Research Institute Chinese Academy of Agricultural Sciences, Lanzhou, Gansu 730000 (CN)
(72) Inventor: ZHENG, Haixue, Lanzhou, Gansu 730000 (CN); RU, Yi, Lanzhou, Gansu 730000 (CN); LIU, Huanan, Lanzhou, Gansu 730000 (CN); GUO, Jianhong, Lanzhou, Gansu 730000 (CN); TIAN, Hong, Lanzhou, Gansu 730000 (CN); CAO, Weijun, Lanzhou, Gansu 730000 (CN); LU, Bingzhou, Lanzhou, Gansu 730000 (CN); YANG, Fan, Lanzhou, Gansu 730000 (CN); SHEN, Chaochao, Lanzhou, Gansu 730000 (CN); ZHANG, Wei, Lanzhou, Gansu 730000 (CN); LI, Yajun, Lanzhou, Gansu 730000 (CN); YANG, Yang, Lanzhou, Gansu 730000 (CN); SHI, Zhengwang, Lanzhou, Gansu 730000 (CN); HE, Jijun, Lanzhou, Gansu 730000 (CN); JIN, Ye, Lanzhou, Gansu 730000 (CN); LI, Dan, Lanzhou, Gansu 730000 (CN); ZHU, Zixiang, Lanzhou, Gansu 730000 (CN); MAO, Ruoqing, Lanzhou, Gansu 730000 (CN); HAO, Rongzeng, Lanzhou, Gansu 730000 (CN); ZHANG, Guicai, Lanzhou, Gansu 730000 (CN); DANG, Wen, Lanzhou, Gansu 730000 (CN); MA, Xusheng, Lanzhou, Gansu 730000 (CN); QIN, Xiaodong, Lanzhou, Gansu 730000 (CN); LIU, Xiangtao, Lanzhou, Gansu 730000 (CN)
(74) Representative: Patent 42
(86) International application number: PCT/CN2024/086558
(87) International publication number: WO 2024/212919

(57) **Abstract**

The present disclosure belongs to the field of biotechnology, and specifically relates to a seven-component antigen African swine fever subunit vaccine. The present disclosure first provides an African swine fever virus antigen protein combination composed of the African swine fever virus P34 protein, P30 protein, P54 protein, A104R protein, C129R protein, X protein, and Y protein. This African swine fever virus antigen protein combination can induce a strong immune response in the host. Furthermore, the present disclosure provides a seven-component antigen African swine fever subunit vaccine including the aforementioned African swine fever virus antigen protein combination. The seven-component antigen African swine fever subunit vaccine exhibits good immunoprotection rates against challenge with the parental virulent African swine fever virus strain, poses no biosafety risks, overcomes the difficulty that existing African swine fever subunit vaccines domestically and internationally cannot provide effective immunoprotection for pigs, and demonstrates the feasibility of the research approach for African swine fever subunit vaccines.

## Description

### TECHNICAL FIELD

The present disclosure belongs to the field of biotechnology, and specifically relates to a seven-component antigen African swine fever subunit vaccine.

### BACKGROUND

African swine fever (ASF) is the "number one killer" threatening the global swine industry, with a prevalence history of over one hundred years. In August 2018, ASF was introduced into China, severely impacting the domestic swine industry and causing unprecedented losses. To date, no commercialized ASF vaccine has been made available, presenting a critical challenge and pain point for the swine industry and constituting a major national demand. Scientific research both domestically and internationally indicates that ASF inactivated vaccines, subunit vaccines, and live vector vaccines still fail to address the issue of immunogenic efficacy. Although gene-deleted vaccines resolve the immunogenic efficacy concern, they pose risks associated with residual virulence and biosafety. The African swine fever virus (ASFV) possesses a large genome encoding over 150 viral proteins, most of which have unclear functions. The antigens responsible for inducing and stimulating the immune system remain poorly understood, which represents a key obstacle in developing an African swine fever vaccine with ideal immunogenic efficacy.

Regarding subunit vaccines, Chinese Patent CN115814071A discloses a subunit vaccine comprising African swine fever virus p34, p14, C129R, DP96R, A104R, p54, p17, p22, P72, and p30 proteins; Chinese Patent CN115702928A discloses a subunit vaccine comprising African swine fever virus p34, p14, C129R, DP96R, A104R, p54, p22, P72, and p30 proteins; Chinese Patent CN112472801A discloses a subunit vaccine comprising African swine fever virus P30 protein, and African swine fever virus P54 membrane structural protein. Although the aforementioned studies have disclosed subunit vaccines with different antigen compositions, none have systematically evaluated the immunogenic efficacy of the vaccines. Further research by the applicant's team has also revealed that certain antigen components involved in the aforementioned subunit vaccines may exhibit antibody-dependent enhancement (ADE) effects, which could severely compromise the protective efficacy of the subunit vaccines against virulent strains of African swine fever. The term "ADE effect" refers to a process wherein virus-specific antibodies bind to the virus, and the virus-antibody complexes subsequently bind via the Fc segment to cells expressing Fc receptors (FcR) on their surface, thereby facilitating viral entry into these cells and enhancing the infectivity of the virus.

Based on the above issues, the applicant provides a seven-component antigen African swine fever subunit vaccine. The subunit vaccine demonstrates ideal protective efficacy against challenge with a parental virulent strain of African swine fever.

### SUMMARY

In response to the above technical problems, the present disclosure, through extensive experimental screening and evaluation research, has unexpectedly discovered a seven-component antigen African swine fever subunit vaccine. The subunit vaccine not only induces a superior immune response and exhibits a favorable safety profile, but also provides ideal protective efficacy upon challenge with a parental virulent strain of African swine fever. Specifically, the present disclosure includes the following content.

In a first aspect, the present disclosure provides an ASFV antigen protein combination, wherein the ASFV antigen protein combination consists of a P34 protein, a P30 protein, a P54 protein, an A104R protein, an X protein, a C129R protein, and a Y protein; wherein the X protein is a DP96R protein or a fusion protein of the DP96R protein and an P12 protein; and the Y protein is a P22 protein, or a P17 protein, or a combination of the P22 protein and the P17 protein, or a fusion protein of the P22 protein and the P17 protein, or a fusion protein of a fragment of the P22 protein and a fragment of the P17 protein.

Preferably, the ASFV is a genotype II ASFV.

Preferably, the genotype II ASFV is ASFV CN/GS 2018.

Preferably, the amino acid sequence of the P34 protein is set forth in SEQ ID NO: 1; the amino acid sequence of the P30 protein is set forth in SEQ ID NO: 3; the amino acid sequence of the P54 protein is set forth in SEQ ID NO: 5 or SEQ ID NO: 7; the amino acid sequence of the A104R protein is set forth in SEQ ID NO: 9; the amino acid sequence of the DP96R protein is set forth in SEQ ID NO: 11; the amino acid sequence of the fusion protein of the DP96R protein and the P12 protein is set forth in SEQ ID NO: 13; the amino acid sequence of the C129R protein is set forth in SEQ ID NO: 15; the amino acid sequence of the P17 protein is set forth in SEQ ID NO: 17; the amino acid sequence of the P22 protein is set forth in SEQ ID NO: 19; the amino acid sequence of the fusion protein of the fragment of the P17 protein and the fragment of the P22 protein is set forth in SEQ ID NO: 21; and the amino acid sequence of the fusion protein of the P17 protein and the P22 protein is set forth in SEQ ID NO: 23.

Preferably, the P34 protein, the P30 protein, the P54 protein, the A104R protein, the X protein, the C129R protein, and the Y protein are in a ratio of (1-6):(1-6):(1-6):(1-6):(1-6):(1-6):(1-6).

Preferably, the P34 protein, the P30 protein, the P54 protein, the A104R protein, the X protein, the C129R protein, and the Y protein are in a ratio of (1-2):1:(1-2):1:(1-2):1:1.

Preferably, the P34 protein, the P30 protein, the P54 protein, the A104R protein, the X protein, the C129R protein, and the Y protein are in a ratio of 2:1:(1-2):1:2:1:1.

Preferably, the P34 protein, the P30 protein, the P54 protein, the A104R protein, the X protein, the C129R protein, and the Y protein are in a ratio of 2:1:1:1:2:1:1.

Preferably, the concentration of each of the P34 protein, the P30 protein, the P54 protein, the A104R protein, the X protein, the C129R protein, and the Y protein is greater than or equal to 50 µg/mL.

Preferably, the concentration of each of the P34 protein, the P30 protein, the P54 protein, the A104R protein, the X protein, the C129R protein, and the Y protein is greater than or equal to 90 µg/mL.

Preferably, the concentration of each of the P34 protein, the P30 protein, the P54 protein, the A104R protein, the X protein, the C129R protein, and the Y protein is greater than or equal to 150 µg/mL.

Preferably, the concentration of each of the P34 protein, the P30 protein, the P54 protein, the A104R protein, the X protein, the C129R protein, and the Y protein is from 150 µg/mL to 2400 µg/mL.

Preferably, the concentrations of the P34 protein, the P30 protein, the P54 protein, the A104R protein, the X protein, the C129R protein, and the Y protein are 400 µg/mL, 200 µg/mL, 200 µg/mL, 200 µg/mL, 400 µg/mL, 200 µg/mL, and 200 µg/mL, respectively.

Preferably, the concentrations of the P34 protein, the P30 protein, the P54 protein, the A104R protein, the X protein, the C129R protein, and the Y protein are 400 µg/mL, 200 µg/mL, 400 µg/mL, 200 µg/mL, 400 µg/mL, 200 µg/mL, and 200 µg/mL, respectively.

Preferably, the P34 protein, the P30 protein, the P54 protein, the A104R protein, the X protein, and the C129R protein are obtained by expression in an *Escherichia coli* system; and the Y protein is obtained by expression in a Chinese hamster ovary (CHO) expression system.

In a second aspect, the present disclosure provides a use of the ASFV antigen protein combination according to the first aspect in the manufacture of a medicament for preventing or treating an ASFV infection.

In a third aspect, the present disclosure provides a use of the ASFV antigen protein combination according to the first aspect in the manufacture of a biological product for preventing an ASFV infection.

In a fourth aspect, the present disclosure provides a seven-component antigen African swine fever subunit vaccine, wherein the seven-component antigen African swine fever subunit vaccine consists of the ASFV antigen protein combination according to the first aspect and a pharmaceutically acceptable adjuvant.

Preferably, the adjuvant comprises one or more selected from the group consisting of: a chemical immunological adjuvant, a microbial immunological adjuvant, a plant-based immunological adjuvant, and a biochemical immunological adjuvant.

The beneficial effects of the present disclosure are as follows: (1) The present disclosure provides a P34 protein, a P30 protein, a P54 protein, an A104R protein, an X protein, a C129R protein, and a Y protein; wherein the X protein is a DP96R protein or a fusion protein of the DP96R protein and an P12 protein; and the Y protein is a P22 protein, or a P17 protein, or a combination of the P22 protein and the P17 protein, or a fusion protein of the P22 protein and the P17 protein; wherein the ASFV antigen protein combination is capable of inducing a favorable immune response; (2) A seven-component subunit vaccine prepared by combining the ASFV antigen protein combination of the present disclosure with a vaccine adjuvant provides good protective efficacy upon challenge with a parental virulent strain of African swine fever; (3) The seven-component subunit vaccine described in the present disclosure exhibits a favorable safety profile; (4) The seven-component subunit vaccine described in the present disclosure has a simple preparation process, is suitable for large-scale industrial production, and possesses significant social and economic value.

### BRIEF DESCRIPTION OF THE DRAWINGS

Fig. 1 shows gene identification results of plasmid pET-A-1#, where M: DL2000 DNA marker; and 1: different plasmids.
Fig. 2 shows gene identification results of plasmid pET-A-2#, where M: DL2000 DNA marker; and 1: different plasmids.
Fig. 3 shows gene identification results of plasmid pET-A-3#, where M: DL2000 DNA marker; and 1: different plasmids.
Fig. 4 shows gene identification results of plasmid pET-A-4#, where M: DL2000 DNA marker; and 1: different plasmids.
Fig. 5 shows gene identification results of plasmid pET-A-6#, where M: DL2000 DNA marker; and 1: different plasmids.
Fig. 6 shows gene identification results of plasmid pET-A-7#, where M: DL2000 DNA marker; and 1: different plasmids.
Fig. 7 shows expression results of P34 protein, where M: protein molecular weight marker; 1: post-lysis supernatant before induction; 2: post-lysis cell pellet before induction; 3: post-lysis supernatant after induction; and 4: post-lysis cell pellet after induction.
Fig. 8 shows expression results of P30 protein, where M: protein molecular weight marker; 1: post-lysis supernatant before induction; 2: post-lysis cell pellet before induction; 3: post-lysis supernatant after induction; and 4: post-lysis cell pellet after induction.
Fig. 9 shows expression results of P54 protein, where M: protein molecular weight marker; 1: post-lysis supernatant before induction; 2: post-lysis cell pellet before induction; 3: post-lysis supernatant after induction; and 4: post-lysis cell pellet after induction.
Fig. 10 shows expression results of A104R protein, where M: protein molecular weight marker; 1: post-lysis supernatant before induction; 2: post-lysis cell pellet before induction; 3: post-lysis supernatant after induction; and 4: post-lysis cell pellet after induction.
Fig. 11 shows expression results of X protein, where M: protein molecular weight marker; 1: post-lysis supernatant before induction; 2: post-lysis cell pellet before induction; 3: post-lysis supernatant after induction; and 4: post-lysis cell pellet after induction.
Fig. 12 shows expression results of C129R protein, where M: protein molecular weight marker; 1: post-lysis supernatant before induction; 2: post-lysis cell pellet before induction; 3: post-lysis supernatant after induction; and 4: post-lysis cell pellet after induction.
Fig. 13 shows Western blot analysis results of the antigens. From left to right are the P34 protein, P30 protein, P54 protein, A104R protein, X protein, and C129R protein; where M: protein molecular weight marker; 1: results of specific reaction between different antigens and ASFV-positive serum.
Fig. 14 shows expression results of the African swine fever virus Y protein. From left to right are: gene identification results of pCHO-A-9# (M: DL10000 DNA marker; 1: target gene expression from the pCHO-A-9# plasmid), protein purification results (M: protein molecular weight marker; 1: CHO supernatant after induction; 2: elution with 50 mM imidazole; 3: elution with 400 mM imidazole), and Western blot analysis results of the antigen (M: protein molecular weight marker; 1: results of specific reaction between the protein and ASFV-positive serum).

### DETAILED DESCRIPTION OF THE EMBODIMENTS

To make the technical means, creative features, objectives, and effects achieved by the present disclosure easily understood, the present disclosure is further described below in conjunction with the specific embodiments. However, the scope of protection of the present disclosure is not limited to the following embodiments.

The experiments described in the following embodiments obtained biosafety approval and ASF laboratory activity approval:

In accordance with the relevant requirements for Biosafety Level 3 laboratories (BSL-3) and African Swine Fever-related biosafety, the Lanzhou Veterinary Research Institute of the Chinese Academy of Agricultural Sciences, upon review and reporting level by level by the Lanzhou Veterinary Research Institute Biosafety Committee, the Lanzhou Veterinary Research Institute Experimental Animal Ethics Committee, the Chinese Academy of Agricultural Sciences Biosafety Committee, the Lanzhou Veterinary Research Institute Experimental Animal Ethics Committee, and the Lanzhou Veterinary Research Institute Biosafety Committee, obtained permission from the Ministry of Agriculture for conducting research on highly pathogenic ASFV pathogens and animals. This research has been filed with the Ministry of Agriculture and Rural Affairs and complies with national biosafety level requirements.

Sources of experimental cells, viruses, and plasmids described in the following embodiments:
BL21 (DE3) competent cells and pET28a expression vectors were purchased from Shanghai Solarbio Biotechnology Co., Ltd. Sf9 insect cells, pFastBac^{™} vectors, and DH10Bac^{™} competent cells were all purchased from Thermo Fisher. P3xflag-cmv-7.1 vectors, pcDNA3.1 vectors, and suspension cultured CHO cells were all deposited by Lanzhou Veterinary Research Institute, Chinese Academy of Agricultural Sciences.

African Swine Fever virus protein expression plasmids were all constructed by classical molecular gene cloning methods, using African Swine Fever virus cDNA as a template, Polymerase Chain Reaction (PCR)-amplifying the coding regions of different viral proteins, and ligating the amplified products into the pCMV-C-Flag vector. Primary porcine alveolar macrophages (PAM) were prepared by the present laboratory (prepared by lavage and separation from pig lungs).

LB liquid medium (dry powder) and LB solid medium (dry powder) were purchased from Solarbio Science & Technology (Beijing) Co., Ltd.; kanamycin, IPTG (Isopropyl β-D-1-thiogalactopyranoside), and X-Gal (5-Bromo-4-chloro-3-indolyl- β -D-galactopyranoside) were purchased from Shanghai Solarbio Technology Co., Ltd.; Gel Extraction Kit (100) and Plasmid Mini Kit I (100) were purchased from OMEGA Bio-tek, USA; conventional molecular and chemical reagents were all purchased from Sinopharm Chemical Reagent Co., Ltd. PureLinK HiPure Plasmid Maxiprep kit, ExpiSf^{™} Protein Production Kit, and ExpiFectamine^{™} CHO Transfection Kit were all purchased from Invitrogen Corporation; DNA Marker was purchased from TaKaRa Biotechnology Co., Ltd.

The genotype II African Swine Fever virus strain ASFV CN/GS 2018 was obtained from the National African Swine Fever Regional Laboratory (Lanzhou), belongs to genotype II, has a virus titer of 1×10⁵ HAD₅₀/mL, is the 4th generation seed virus amplified in PAM cells, and was deposited on December 21, 2020, at the China Center for Type Culture Collection (CCTCC) under accession number CCTCC NO: V202096. The deposition address is: Wuhan University, Wuhan, China; contact telephone: 027-68752319.

Based on the viral protein gene sequences encoded by the ASFV CN/GS/2018 isolate, coding region fragments for different viral proteins were designed and synthesized, and eukaryotic expression vectors were constructed. The expression sequences for each gene were designed through codon optimization (including eliminating rare codons and adjusting GC content, etc.). The optimized gene sequences were sent to a company for synthesis, and prokaryotic expression vectors were constructed.

Experimental rabbits: Healthy male New Zealand rabbits, approximately 2.0 kg, purchased from the Experimental Animal Center of Lanzhou Veterinary Research Institute, Chinese Academy of Agricultural Sciences.

Pigs: Healthy pigs, all purchased from pig farms without ASFV outbreaks, testing negative for PCV2 antigen, and testing negative for ASFV and PRRSV antigens and antibodies.

### Definitions:

The term "molecular chaperone" refers to a class of proteins that are unrelated in sequence but share a common function. These proteins assist other polypeptide-containing structures within the cell to achieve correct assembly and dissociate from these structures after assembly is complete, without becoming a component of these protein structures when they perform their functions.

The term "truncated protein" refers to a protein from which the transmembrane domain has been removed, or to an extracellular domain protein.

The term "antigen" refers to a substance capable of inducing an immune response in an organism. Specifically, the antigen can be specifically recognized and bound by the antigen receptors (TCR/BCR) on the surfaces of T or B lymphocytes, activate T or B cells, cause their proliferation and differentiation, produce immune response products (sensitized lymphocytes or antibodies), and can specifically bind with the corresponding products *in vivo* or *in vitro.*

The terms "vaccine," "vaccine composition," and "subunit vaccine" refer to a pharmaceutical composition containing an African Swine Fever virus protein antigen. This pharmaceutical composition can induce, stimulate, or enhance an immune response in pigs against African Swine Fever, and is a biological preparation capable of providing a protective response in animals, wherein the vaccine has been administered and does not cause severe disease.

Further, the "vaccine," "vaccine composition," or "subunit vaccine" comprises one or more adjuvants, excipients, carriers, and diluents.

Further, the adjuvant comprises one or more selected from the group consisting of chemical immunological adjuvants, microbial immunological adjuvants, plant-based immunological adjuvants, and biochemical immunological adjuvants.

Further, the chemical immunological adjuvants include aluminum hydroxide, Freund's adjuvant, mineral oil, Span, and the like; the microbial immunological adjuvants include Mycobacterium, lipopolysaccharide, muramyl dipeptide, cytosine-phosphate-guanine (CpG), lipid-soluble Wax D, Corynebacterium parvum (CP); the plant-based immunological adjuvants include polysaccharides extracted from plants or macrofungi, such as Poria cocos polysaccharide, Carthamus tinctorius polysaccharide, Chinese herbal medicines, and the like; the biochemical immunological adjuvants include thymosin, transfer factor, interleukin, and the like. Preferred adjuvants may be nano-adjuvants, biological adjuvants, interleukin, interferon, and the like.

Further, the "vaccine," "vaccine composition," or "subunit vaccine" may also be used in the preparation of combination vaccines, such as combinations with other vaccines for pigs.

Further, administration of the "vaccine," "vaccine composition," or "subunit vaccine" may employ convenient routes, such as intramuscular injection, intranasal, oral, subcutaneous, transdermal, and vaginal routes. The "vaccine," "vaccine composition," or "subunit vaccine" may be administered following a prime-boost regimen; for example, after a first inoculation, a subject may receive a second, booster administration after a period of time (e.g., approximately 7, 14, 21, or 28 days). Typically, the dose for the booster administration is the same as or lower than the dose used for the prime immunization. Additionally, a third booster immunization may be administered, for example, at 2-3 months, 6 months, or one year after immunization.

The term "prevention," in the context of African Swine Fever virus infection, refers to inhibiting replication of the African Swine Fever virus, inhibiting transmission of the African Swine Fever virus, or preventing the African Swine Fever virus from establishing itself within its host, as well as alleviating symptoms of the disease or condition caused by African Swine Fever virus infection.

This experiment first systematically evaluated the immune efficacy of 164 proteins of the genotype II ASFV. Effective ASFV immunogens were screened based on two aspects: host immune response and inhibition of viral replication. (1) Innate immune response: 164 eukaryotic expression plasmids containing different open reading frames from the ASFV genome were constructed. Using dual-luciferase reporter systems for different innate immune pathways, 17 proteins that promote innate immune responses were screened out. (2) Lymphocyte immune response: 120 kinds of ASFV antigen proteins were prepared, from which 23 proteins capable of promoting ASFV-specific lymphocyte proliferation were screened out. (3) Inhibition of viral replication: The top 5 virus proteins promoting the strongest innate immune response and the 23 virus proteins demonstrating significant ability to promote lymphocyte proliferation, totaling 28 proteins from the above screenings, were selected. Proteins obtained from the aforementioned three screening strategies were randomly and directionally combined to obtain a series of multi-component subunit vaccines. Three immunization experiments were conducted in rabbits (New Zealand male rabbits) to prepare immune sera. Through viral erythrocyte adsorption and replication inhibition assays, it was ultimately screened that antibodies against 7 kinds of virus proteins could significantly inhibit viral proliferation. The preparation process of the seven-component subunit vaccine and the immune efficacy evaluation experiments are described below.

### Example 1: Expression of the Seven-Component Antigen Proteins

### 1. Expression of African Swine Fever Virus P34, P30, P54, A104R, X, and C129R Proteins

Based on the P34, P30, P54, A104R, X (which is the DP96R protein or a fusion protein of DP96R protein and P12 protein), and C129R genes of the ASFV CN/GS/2018 isolate, the sequences were optimized and designed to construct recombinant protein expression engineering bacteria. The codon-optimized (including eliminating rare codons and adjusting GC content, etc.) P34, P30, P54, A104R, and C129R genes were respectively cloned into the *Escherichia coli* expression vector pET28a. The X gene was cloned into the *Escherichia coli* expression vector pET32a. The obtained positive plasmids were respectively named pET-A-1#, pET-A-2#, pET-A-3#, pET-A-4#, pET-A-7#, and pET-A-6#. These positive plasmids were respectively transformed into *Escherichia coli* BL21 (DE3) competent cells, obtaining recombinant engineering bacteria capable of expressing P34, P30, P54, A104R, C129R, and X proteins, named E/pET-A-1#, E/pET-A-2#, E/pET-A-3#, E/pET-A-4#, E/pET-A-7#, and E/pET-A-6#. Wherein the amino acid sequence of the P34 protein is set forth in SEQ ID NO: 1, and the encoded gene sequence is set forth in SEQ ID NO: 2; the amino acid sequence of the P30 protein is set forth in SEQ ID NO: 3, and the encoded gene sequence is set forth in SEQ ID NO: 4; the amino acid sequence of the P54 protein is set forth in SEQ ID NO: 7, and the encoded gene sequence is set forth in SEQ ID NO: 8 (for the purpose of facilitating expression, the present disclosure used a truncated form of the P54 protein (with the transmembrane domain removed), the amino acid sequence of which is set forth in SEQ ID NO: 5, and the encoded gene sequence is set forth in SEQ ID NO: 6); the amino acid sequence of the A104R protein is set forth in SEQ ID NO: 9, and the encoded gene sequence is set forth in SEQ ID NO: 10; the X protein is the DP96R protein, the amino acid sequence of which is set forth in SEQ ID NO: 11, and the encoded gene sequence is set forth in SEQ ID NO: 12 (in the present disclosure, the P12 protein was fused during expression of the DP96R protein, the amino acid sequence of which is set forth in SEQ ID NO: 13, and the encoded gene sequence is set forth in SEQ ID NO: 14); the amino acid sequence of the C129R protein is set forth in SEQ ID NO: 15, and the encoded gene sequence is set forth in SEQ ID NO: 16.

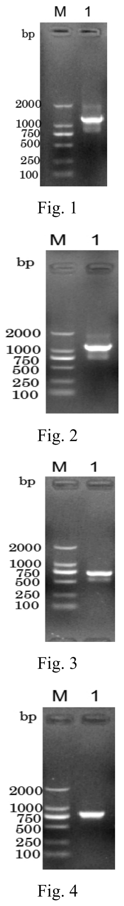

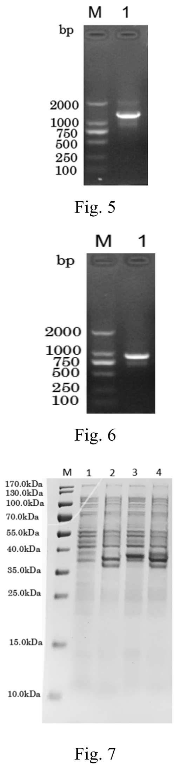

Plasmids were extracted from the recombinant engineering bacteria strains for PCR identification. The pET-A-1# plasmid amplification yielded a band of approximately 1150 base pairs (bp), consistent with the expected fragment size (as shown in Fig. 1). The pET-A-2# plasmid amplification yielded a band of approximately 1200 bp, consistent with the expected fragment size (as shown in Fig. 2). The pET-A-3# plasmid amplification yielded a band of approximately 700 bp, consistent with the expected fragment size (as shown in Fig. 3). The pET-A-4# plasmid amplification yielded a band of approximately 840 bp, consistent with the expected fragment size (as shown in Fig. 4). The pET-A-6# plasmid amplification yielded a band of approximately 1100 bp, consistent with the expected fragment size (as shown in Fig. 5). The pET-A-7# plasmid amplification yielded a band of approximately 900 bp, consistent with the expected fragment size (as shown in Fig. 6).

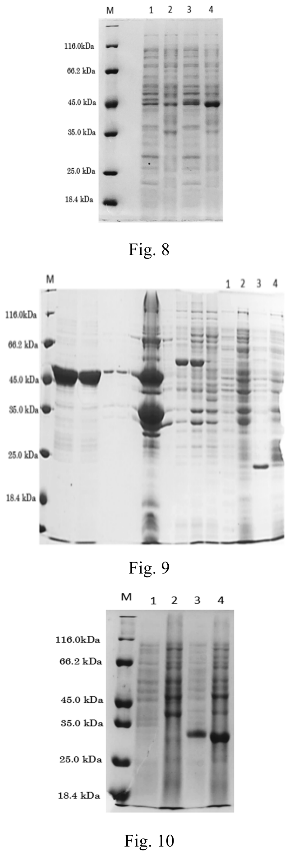

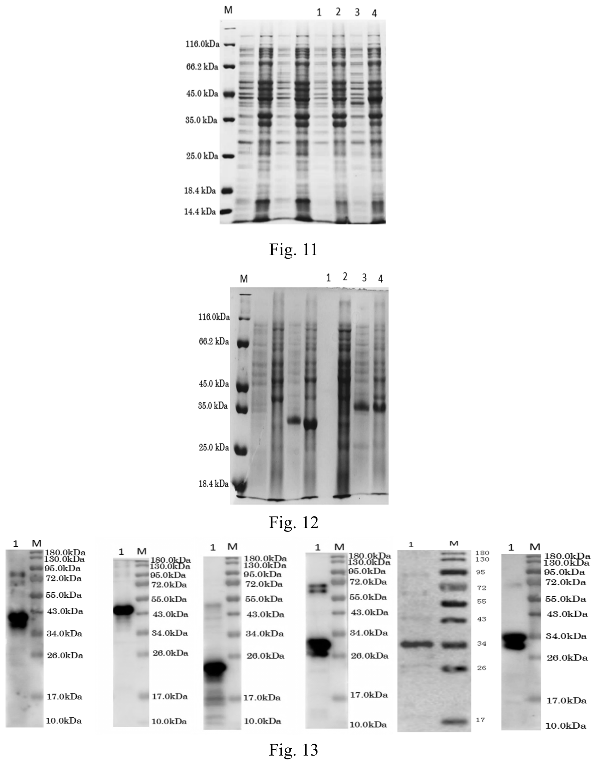

These recombinant bacterial strains, upon induction by IPTG, expressed soluble P34 protein (as shown in Fig. 7), P30 protein (as shown in Fig. 8), P54 protein (as shown in Fig. 9), A104R protein (as shown in Fig. 10), DP96R protein (as shown in Fig. 11), and C129R protein (as shown in Fig. 12), with sizes of approximately 37 kilodaltons (kDa), 45 kDa, 24 kDa, 31 kDa, 35 kDa, and 34 kDa, respectively. Western blot analysis confirmed that these proteins could react with African Swine Fever virus-positive serum (results as shown in Fig. 13).

### 2. Expression of African Swine Fever Virus Y protein

The Y protein is the P22 protein (amino acid sequence set forth in SEQ ID NO: 17, gene sequence set forth in SEQ ID NO: 18), or the P17 protein (amino acid sequence set forth in SEQ ID NO: 19, gene sequence set forth in SEQ ID NO: 20), or a combination of the P22 protein and the P17 protein, or a fusion protein of a fragment of the P22 protein and a fragment of the P17 protein (a fusion protein comprising the extracellular domain of the P22 protein and the extracellular domain of the P17 protein, amino acid sequence set forth in SEQ ID NO: 21, gene sequence set forth in SEQ ID NO: 22), or a fusion protein of the P22 protein and the P17 protein (amino acid sequence set forth in SEQ ID NO: 23, gene sequence set forth in SEQ ID NO: 24). This embodiment uses the fusion protein of the extracellular domain of the P22 protein and the extracellular domain of the P17 protein as an example for expressing the Y protein, as detailed below:

Based on the P22 protein and P17 protein genes of the ASFV CN/GS/2018 isolate, the sequence was optimized and designed to construct a stable CHO cell line. The codon-optimized P17-P22 gene (SEQ ID NO: 22), which encodes the fusion protein of the extracellular domain of the P22 protein and the extracellular domain of the P17 protein, was cloned into the eukaryotic expression vector pCHO1.0. The resulting recombinant expression plasmid was named pC1.0-A-9#. This plasmid was transfected into CHO cells. Following selection with puromycin and methotrexate, a recombinant CHO cell line capable of stably expressing the Y protein was obtained and named pCHO-A-9#.

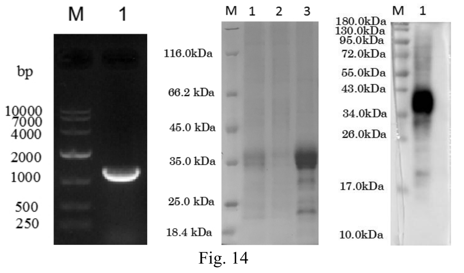

The results are shown in Fig. 14. Genomic DNA extracted from the pCHO-A-9# cell line was subjected to PCR identification, yielding a band of approximately 1400 bp, consistent with the expected fragment size. The recombinant CHO cell line secreted and expressed the Y protein in the culture supernatant, with a size of approximately 38 kDa. Western blot analysis confirmed that the Y protein could react with African Swine Fever virus-positive serum. A person skilled in the art can similarly use the method described above to express the P22 protein alone, the P17 protein alone, or a fusion protein of the fragment of the P22 protein and the fragment of the P17 protein.

Although the present disclosure specifies particular expression methods for the seven-component proteins, the scope is not limited to these methods. A person skilled in the art can express and purify the seven-component antigen proteins described in the present disclosure using conventional expression systems. The expression host is not limited to prokaryotic expression systems such as *Escherichia coli* or Bacillus subtilis, nor is it limited to eukaryotic expression systems such as insect cells, mammalian cells, or yeast cells. The expression vector is not limited to the aforementioned pET28a, pFastBac, or pCHO1.0. A person skilled in the art can also select conventional plasmids based on the chosen expression system, including prokaryotic expression plasmids known in the art such as the pET series, pET-GST, pGEX series, pMAL series, pBAD series, pQE series, pCold series, etc.; and eukaryotic expression plasmids such as flashBAC series, pEGFP, pCDM8, pCNVp-NEO-BAN, pEGFT-Actin, pSV2, CMV4, etc.

In the present disclosure, when expressing the aforementioned seven-component proteins, the sequences of the antigen proteins described above can also be optimized using methods including truncation, mutation, introduction of fusion tags, or introduction of chaperone proteins to enhance the expression of soluble target protein and purification efficiency.

The truncation strategy involves removing the transmembrane domain of the target protein, removing localization signals, removing specific domains, etc.

The chaperone protein can be selected from one or a combination of several of the following: DnaK protein, DnaJ protein, GroEL protein, GroES protein, GrpE protein, HSP (Heat Shock Protein), *Trigger Factor protein,* etc.

The fusion tag is selected from one or a combination of several of the following: SUMO (Small Ubiquitin-like Modifier) protein, GST (Glutathione S-Transferase) protein, Trx (Thioredoxin) protein, NusA (N-utilization substance A) protein, DsbA (Disulfide bond formation protein A) protein, DsbC (Disulfide bond formation protein C) protein, MBP (Maltose-Binding Protein) protein, etc.

### Example 2 Immunogenicity Test of Immunogenicity of African Swine Fever Virus Antigen Protein Combination

### 1. Rabbit Immunization Experiment

The seven antigen protein components (designated as 1#, 2#, 3#, 4#, 6#, 7#, and 9#, representing the P34 protein, P30 protein, P54 protein, A104R protein, X protein, C129R protein, and Y protein, respectively) expressed and purified as described above were each thoroughly mixed and emulsified with an equal volume of Freund's Complete Adjuvant (FCA). Twenty-one healthy male rabbits, each weighing approximately 2.0 kg, were selected. After a two-day acclimatization period, three rabbits were immunized for each emulsified antigen via subcutaneous multi-point injections along the back. The immunization dose was 300 µg per rabbit. Booster immunizations were administered on day 15 and day 30 after the primary immunization using an equal amount of protein emulsified with Freund's Incomplete Adjuvant (FIA), following the same injection sites and method as the primary immunization. Fourteen days after the third immunization, a small amount of blood was collected from the ear vein, serum was separated, and the antibody titer was determined using an indirect Enzyme-Linked Immunosorbent Assay (ELISA) method. The maximum serum dilution factor yielding a Signal-to-Noise (S/N) ratio ≥ 2.1 was defined as the antibody titer. If the serum titer reached at least 1:64000, blood was collected routinely, serum was separated, and stored at -20°C or below. If the immune serum titer was low, an additional booster immunization was administered. The antibody titer was re-assessed 14 days later. If the serum titer met the requirement, serum was prepared as described above. Otherwise, the serum was discarded and preparation was repeated.

Each of the seven antigen components was diluted with coating buffer to a concentration of 1 µg/mL. Then, 100 µL per well of each diluted antigen was added to separate ELISA plates. The plates were coated at 2-8°C for 16 hours. After coating, the plates were washed twice with wash buffer, and the residual liquid was removed by flicking and blotting dry. A blocking solution (150 µL/well) was added, and the plates were blocked at 37°C for 2 hours. After blocking, the liquid was removed by flicking, the plates were blotted dry, and then air-dried in a dry, ventilated environment. The dried plates were vacuum-sealed in aluminum foil bags containing desiccant and stored at 4°C for future use.

To determine the antibody titer of the polyclonal antiserum from rabbits immunized with each protein, the prepared rabbit antiserum was serially diluted with Phosphate Buffered Saline (PBS) at ratios of 1:1000, 1:2000, 1:4000, 1:8000, 1:16000, 1:32000, 1:64000, and 1:128000. The rabbit antibody titer was detected using the indirect ELISA method. Serum from non-immunized rabbits was used as a negative control. The specific criterion for a positive result was an S/N ratio ≥ 2.1 (where S represents the OD₄₅₀ₙₘ value of the sample and N represents the OD₄₅₀ₙₘ value of the negative control). The maximum dilution factor of the rabbit antiserum meeting this criterion was recorded as the rabbit antibody titer.

Fourteen days after the third immunization, blood was collected from the rabbit ear vein, serum was separated, and ELISA titer detection was performed. The results demonstrated that the antibody titers for all seven antigen components were greater than 1:64000.

### 2. Mouse Immunization Experiment

The seven antigen protein components (designated as 1#, 2#, 3#, 4#, 6#, 7#, and 9#, representing the P34 protein, P30 protein, P54 protein, A104R protein, X protein, C129R protein, and Y protein, respectively) expressed and purified as described above were each diluted with sterile PBS to a concentration of 300 µg/mL. For each protein, 1.5 mL of the recombinant protein solution was thoroughly mixed and emulsified with an equal volume of FCA. Three healthy six-week-old female mice were selected. After a two-day acclimatization period, each mouse received a 200 µL subcutaneous multi-point injection along the back of the emulsified recombinant protein.

Booster immunizations were administered on day 15 and day 30 after the primary immunization using an equal amount of protein emulsified with FIA, following the same injection sites and method as the primary immunization. Fifteen days after the third immunization, blood was collected from the tail vein, serum was separated, and the antibody level of each mouse was determined using the indirect ELISA method. Splenocytes from mice whose serum, even at a 1:16000 dilution, still yielded an OD₄₅₀ₙₘ value greater than 1.0 were selected for cell fusion. The mice designated for fusion received a final booster immunization three days prior to the fusion procedure. This booster was administered via intraperitoneal injection of 0.5 mL of recombinant protein solution (300 µg/mL) without adjuvant.

Fifteen days after the third immunization, blood was collected from the mouse tail vein, serum was separated, and ELISA was performed, recording the OD450nm values. The results indicated that when the serum was diluted to 1:16,000, the OD450nm values of the sera from mice immunized with each of the seven recombinant protein components were all greater than 1.0. Detailed results are presented in Table 1.

**Table 1: ELISA Results of Mouse Sera 15 Days After the Third Immunization with the Seven Recombinant Protein Components**

| Recombinant Protein | Sample No. | Serum Dilution Factor | | | |
|---|---|---|---|---|---|
| | | 1:2000 | 1:4000 | 1:8000 | 1:16000 |
| Negative Control | Control | 0.152 | 0.135 | 0.127 | 0.106 |
| | 1#1 | 2.901 | 2.917 | 2.978 | 2.089 |
| 1# | 1#2 | 3.004 | 2.864 | 2.072 | 1.526 |
| | 1#3 | 2.969 | 3.031 | 2.068 | 1.312 |
| 2# | 2#1 | 2.708 | 2.850 | 2.462 | 1.429 |
| | 2#2 | 2.646 | 2.899 | 2.093 | 1.656 |
| | 2#3 | 2.844 | 2.927 | 2.671 | 1.865 |
| 3# | 3#1 | 2.849 | 2.879 | 2.232 | 2.288 |
| | 3#2 | 3.433 | 2.889 | 2.542 | 1.255 |
| | 3#3 | 2.666 | 2.915 | 2.669 | 1.424 |
| 4# | 4#1 | 3.366 | 2.926 | 2.550 | 1.924 |
| | 4#2 | 3.373 | 2.809 | 2.138 | 1.424 |
| | 4#3 | 2.791 | 2.881 | 2.144 | 2.066 |
| 6# | 6#1 | 3.108 | 3.050 | 2.862 | 1.629 |
| | 6#2 | 2.846 | 3.099 | 2.493 | 1.356 |
| | 6#3 | 3.044 | 3.127 | 2.871 | 2.265 |
| 7# | 7#1 | 3.049 | 3.279 | 2.632 | 2.488 |
| | 7#2 | 3.833 | 3.289 | 2.942 | 1.455 |
| | 7#3 | 3.066 | 3.315 | 3.069 | 1.824 |
| 9# | 9#1 | 2.766 | 3.015 | 2.769 | 1.424 |
| | 9#2 | 3.566 | 3.026 | 2.650 | 1.924 |
| | 9#3 | 3.473 | 2.909 | 2.238 | 1.524 |

The above results indicate that the African Swine Fever virus antigen protein combination provided by the present disclosure, composed of the P34 protein, P30 protein, P54 protein, A104R protein, X protein, C129R protein, and Y protein, is capable of eliciting a robust immune response.

### Example 3 Immunoprotection Test of the Seven-Component Antigen African Swine Fever Subunit Vaccine

### 1. Preparation of the Seven-Component Antigen African Swine Fever Subunit Vaccine

First, seven-component antigen protein combinations with different ratios were obtained according to Table 2. Then, the seven-omponent antigen protein combinations with different ratios were each thoroughly mixed and emulsified with an equal volume of ISA 201 adjuvant (Seppic, France) to prepare African Swine Fever virus seven-component subunit vaccines 1 through 11.

**Table 2: Formulation of the Seven-Component Antigen African Swine Fever Subunit Vaccine**

| Protein | P34 (µg/mL) | P30 (µg/mL) | P54 (µg/mL) | A104R (µg/mL) | X (µg/mL) | C129R (µg/mL) | Y (µg/mL) | Adjuvant (%) |
|---|---|---|---|---|---|---|---|---|
| Vaccine 1 | 600 | 600 | 600 | 600 | 600 | 600 | 600 | 50 |
| Vaccine 2 | 300 | 300 | 300 | 300 | 300 | 300 | 300 | 50 |
| Vaccine 3 | 200 | 200 | 200 | 200 | 200 | 200 | 200 | 50 |
| Vaccine 4 | 2400 | 2400 | 2400 | 2400 | 2400 | 2400 | 2400 | 50 |
| Vaccine 5 | 90 | 90 | 90 | 90 | 90 | 90 | 90 | 50 |
| Vaccine 6 | 150 | 150 | 150 | 150 | 150 | 150 | 150 | 50 |
| Vaccine 7 | 200 | 200 | 400 | 200 | 400 | 200 | 200 | 50 |
| Vaccine 8 | 400 | 200 | 400 | 200 | 400 | 200 | 200 | 50 |
| Vaccine 9 | 400 | 200 | 200 | 200 | 200 | 200 | 200 | 50 |
| Vaccine 10 | 400 | 200 | 200 | 200 | 400 | 200 | 200 | 50 |
| Vaccine 11 | 1200 | 200 | 200 | 200 | 1200 | 200 | 200 | 50 |

### 2. Immunization Experiment

Detection Kits: The African Swine Fever Virus Fluorescent PCR Detection Kit, the African Swine Fever Virus Blocking ELISA Antibody Detection Kit, and the Porcine Circovirus Type II Direct Amplification Fluorescent Quantitative PCR Antigen Detection Kit were provided by the Lanzhou Veterinary Research Institute of the Chinese Academy of Agricultural Sciences. The RealPCR PRRSV-2 RNA Detection Premix and the Porcine Reproductive and Respiratory Syndrome Virus Antibody X3 Detection Kit were purchased from IDEXX Laboratories, Inc.

Challenge Virus: The ASFV CN/GS/2018 strain, batch numbers ASFV CN/GS/2018/1901 and ASFV CN/GS/2018/2001, was provided by the Lanzhou Veterinary Research Institute of the Chinese Academy of Agricultural Sciences.

Experimental Animals: Test pigs aged 60-75 days were all purchased from pig farms with no history of ASFV, PRRSV, or PCV-2 outbreaks. The pigs tested negative for ASFV and PRRSV antigens and antibodies, and tested negative for PCV-2 antigen.

### 2.1 Animal Immunization

The prepared seven-component subunit vaccines 1-11 described above were used to immunize test pigs aged 60-75 days. Each vaccine was administered to 5-10 pigs via intramuscular injection behind the ear root, at a dose of 2-3 mL per pig (varying in antigen composition and content). On day 21 after the primary immunization, a booster immunization was administered via intramuscular injection behind the opposite ear, at a dose of 2-3 mL per pig (varying in antigen composition and content). A control group of 5 unimmunized pigs was established concurrently.

### 2.2 Virus Challenge Evaluation

Fourteen days after the booster immunization, the pigs were challenged with the ASFV CN/GS/2018 strain for evaluation. The challenge dose was 1.25 HAD50/3 mL per pig (equivalent to 5 MLD). After challenge, the pigs were observed continuously for 21 days. Rectal temperature was measured daily, and clinical symptoms were recorded. All control pigs developed disease, and 5 out of 5 died within the experimental period, indicating successful establishment of the challenge model. Immunized pigs were considered protected if they met all the following criteria: rectal temperature did not exceed 40.5°C; if rectal temperature was ≥ 40.5°C, the fever persisted for no more than 2 days; no typical clinical symptoms caused by ASFV infection (such as depression, decreased appetite or anorexia, vomiting, cyanosis of both ears or the entire body skin, bleeding from natural orifices) appeared; and no death caused by ASFV infection occurred during the experimental period. The vaccine protection rate was calculated based on the number of protected immunized pigs.

### 2.3 Sample Collection

On days 5, 10, 15, and 21 post-challenge, 5 mL of blood was collected from each pig (4 mL for serum separation, 1 mL for preparing anticoagulated blood using EDTA-K2). Anal swab samples were also collected simultaneously. All samples were stored at -70°C or below.

### 3. Results

### 3.1 Immunoprotective Efficacy of Seven-Component Subunit Vaccines 1-11

The immunoprotective efficacy results for the seven-component subunit vaccines 1-11 are shown in Table 3. The results indicate that the seven-component vaccine described in the present disclosure provides good protective efficacy against challenge with the parental virulent strain.

**Table 3: Challenge Protection Ratio of Different Vaccines**

| Vaccine No. | Challenge Protection Ratio |
|---|---|
| Vaccine 1 | 7/10 |
| Vaccine 2 | 7/10 |
| Vaccine 3 | 6/10 |
| Vaccine 4 | 5/5 |
| Vaccine 5 | 3/5 |
| Vaccine 6 | 6/10 |
| Vaccine 7 | 4/5 |
| Vaccine 8 | 5/5 |
| Vaccine 9 | 8/10 |
| Vaccine 10 | 10/10 |
| Vaccine 11 | 5/5 |

### 3.2 Body Temperature Monitoring and Clinical Symptom Observation in Test Pigs Post-Challenge

The immunized and protected test pigs exhibited normal body temperature throughout the experimental period, or a body temperature ≥ 40.5°C that persisted for no more than two days. These pigs remained healthy and did not show typical clinical symptoms associated with African Swine Fever virus infection, such as depression, decreased appetite or anorexia, vomiting, cyanosis of the ears or the entire body skin, or bleeding from natural orifices. In contrast, immunized but unprotected pigs began to develop fever from day 3 post-challenge, with body temperatures reaching above 41°C, and displayed typical clinical signs of African Swine Fever. All unprotected immunized pigs died between day 7 and day 16 post-challenge. All pigs in the control group began to develop fever from day 3 post-challenge, with body temperatures reaching above 41°C, and died between day 8 and day 15 post-challenge.

### 3.3 Nucleic Acid Test Results in Test Pigs Post-Challenge

ASFV CN/GS/2018 was not detected in the immunized and protected test pigs during the experimental period.

It should be noted that each component in the seven-component subunit vaccine described in the present disclosure, when present at a concentration greater than or equal to 50 µg/mL, provides desirable protective efficacy.

The above results indicate that: (1) The present disclosure provides a P34 protein, a P30 protein, a P54 protein, an A104R protein, an X protein, a C129R protein, and a Y protein; wherein the X protein is a DP96R protein or a fusion protein of the DP96R protein and an P12 protein; and the Y protein is a P22 protein, or a P17 protein, or a combination of the P22 protein and the P17 protein, or a fusion protein of the P22 protein and the P17 protein; and the ASFV antigen protein combination is capable of inducing a favorable immune response; (2) A seven-component subunit vaccine prepared by combining the ASFV antigen protein combination of the present disclosure with a vaccine adjuvant provides good protective efficacy upon challenge with a parental virulent strain of African swine fever; (3) The seven-component subunit vaccine described in the present disclosure exhibits a favorable safety profile; (4) The seven-component subunit vaccine described in the present disclosure has a simple preparation process and is suitable for large-scale industrial production.

## Claims

1. An African swine fever virus (ASFV) antigen protein combination, wherein the ASFV antigen protein combination consists of a P34 protein, a P30 protein, a P54 protein, an A104R protein, an X protein, a C129R protein, and a Y protein; wherein the X protein is a DP96R protein or a fusion protein of the DP96R protein and a P12 protein; and the Y protein is a P22 protein, or a P17 protein, or a combination of the P22 protein and the P17 protein, or a fusion protein of the P22 protein and the P17 protein, or a fusion protein of a fragment of the P22 protein and a fragment of the P17 protein.

2. The ASFV antigen protein combination according to claim 1, wherein the ASFV is a genotype II ASFV.

3. The ASFV antigen protein combination according to claim 2, wherein the genotype II ASFV is ASFV CN/GS 2018.

4. The ASFV antigen protein combination according to claim 3, wherein the amino acid sequence of the P34 protein is set forth in SEQ ID NO: 1; the amino acid sequence of the P30 protein is set forth in SEQ ID NO: 3; the amino acid sequence of the P54 protein is set forth in SEQ ID NO: 5 or SEQ ID NO: 7; the amino acid sequence of the A104R protein is set forth in SEQ ID NO: 9; the amino acid sequence of the DP96R protein is set forth in SEQ ID NO: 11; the amino acid sequence of the fusion protein of the DP96R protein and the P12 protein is set forth in SEQ ID NO: 13; the amino acid sequence of the C129R protein is set forth in SEQ ID NO: 15; the amino acid sequence of the P17 protein is set forth in SEQ ID NO: 17; the amino acid sequence of the P22 protein is set forth in SEQ ID NO: 19; the amino acid sequence of the fusion protein of the fragment of the P17 protein and the fragment of the P22 protein is set forth in SEQ ID NO: 21; and the amino acid sequence of the fusion protein of the P17 protein and the P22 protein is set forth in SEQ ID NO: 23.

5. The ASFV antigen protein combination according to claim 1, wherein the P34 protein, the P30 protein, the P54 protein, the A104R protein, the X protein, the C129R protein, and the Y protein are in a ratio of (1-6):(1-6):(1-6):(1-6):(1-6):(1-6):(1-6).

6. The ASFV antigen protein combination according to claim 5, wherein the P34 protein, the P30 protein, the P54 protein, the A104R protein, the X protein, the C129R protein, and the Y protein are in a ratio of (1-2):1:(1-2):1:(1-2):1:1.

7. The ASFV antigen protein combination according to claim 6, wherein the P34 protein, the P30 protein, the P54 protein, the A104R protein, the X protein, the C129R protein, and the Y protein are in a ratio of 2:1:(1-2):1:2:1:1.

8. The ASFV antigen protein combination according to claim 1, wherein the concentration of each of the P34 protein, the P30 protein, the P54 protein, the A104R protein, the X protein, the C129R protein, and the Y protein is greater than or equal to 50 µg/mL.

9. The ASFV antigen protein combination according to claim 8, wherein the concentration of each of the P34 protein, the P30 protein, the P54 protein, the A104R protein, the X protein, the C129R protein, and the Y protein is greater than or equal to 90 µg/mL.

10. The ASFV antigen protein combination according to claim 9, wherein the concentration of each of the P34 protein, the P30 protein, the P54 protein, the A104R protein, the X protein, the C129R protein, and the Y protein is greater than or equal to 150 µg/mL.

11. The ASFV antigen protein combination according to claim 10, wherein the concentration of each of the P34 protein, the P30 protein, the P54 protein, the A104R protein, the X protein, the C129R protein, and the Y protein is from 150 µg/mL to 2400 µg/mL.

12. The ASFV antigen protein combination according to claim 11, wherein the concentrations of the P34 protein, the P30 protein, the P54 protein, the A104R protein, the X protein, the C129R protein, and the Y protein are 400 µg/mL, 200 µg/mL, 200 µg/mL, 200 µg/mL, 400 µg/mL, 200 µg/mL, and 200 µg/mL, respectively.

13. The ASFV antigen protein combination according to claim 11, wherein the concentrations of the P34 protein, the P30 protein, the P54 protein, the A104R protein, the X protein, the C129R protein, and the Y protein are 400 µg/mL, 200 µg/mL, 400 µg/mL, 200 µg/mL, 400 µg/mL, 200 µg/mL, and 200 µg/mL, respectively.

14. The ASFV antigen protein combination according to claim 11, wherein the P34 protein, the P30 protein, the P54 protein, the A104R protein, the X protein, and the C129R protein are obtained by expression in an *Escherichia coli* system; and the Y protein is obtained by expression in a Chinese hamster ovary (CHO) expression system.

15. A use of the ASFV antigen protein combination according to any one of claims 1-14 in the manufacture of a medicament for preventing or treating an ASFV infection.

16. A use of the ASFV antigen protein combination according to any one of claims 1-14 in the manufacture of a biological product for preventing an ASFV infection.

17. A seven-component antigen African swine fever subunit vaccine, wherein the seven-component antigen African swine fever subunit vaccine consists of the ASFV antigen protein combination according to any one of claims 1-14 and a pharmaceutically acceptable adjuvant.

18. The seven-component antigen African swine fever subunit vaccine according to claim 17, wherein the adjuvant comprises one or more selected from the group consisting of: a chemical immunological adjuvant, a microbial immunological adjuvant, a plant-based immunological adjuvant, and a biochemical immunological adjuvant.
